**Europäisches Patentamt**

**European Patent Office**

**Office européen des brevets**

(19)

(11) Publication number: **0 251 467**
**A2**

(12)

# EUROPEAN PATENT APPLICATION

(21) Application number: **87304436.6**

(22) Date of filing: **19.05.87**

(51) Int. Cl.³: **C 12 N 15/00**
**C 07 K 15/04, G 01 N 33/569**
**A 61 K 39/15, C 07 K 15/06**

(30) Priority: **20.06.86 US 876518**

(43) Date of publication of application:
**07.01.88 Bulletin 88/1**

(84) Designated Contracting States:
**AT BE CH DE ES FR GB IT LI NL**

(71) Applicant: **ABBOTT LABORATORIES**
**14th Street and Sheridan Road North St**
**North Chicago Illinois 60064(US)**

(72) Inventor: **Smith, Robert E.**
**36567 N. Grandwood Drive**
**Gurnee Illinois 60031(US)**

(72) Inventor: **McGonigal, Thomas**
**33720 Royal Oak Lane Apt. 106**
**Wildwood Illinois 60030(US)**

(74) Representative: **Hayward, Denis Edward Peter et al,**
**Lloyd Wise, Tregear & Co. Norman House 105-109 Strand**
**London WC2R 0AE(GB)**

(54) Compositions and methods of use for a major outer capsid protein (VP7) of rotavirus SA-11.

(57) The gene coding for SA-11 rotavirus major outer capsid protein (VP7) was cloned and expressed in a baculovirus-insect cell expression system. The protein, which is antigenically similar to native VP7 protein, has a molecular weight of 34 kilodaltons and is secreted into the tissue culture media. The recombinant VP7 protein is reactive with neutralizing antiserum prepared against SA-11 virus and can be utilized in a vaccine, as an antigenic preparation for producing antibodies or in a diagnostic assay.

## COMPOSITIONS AND METHODS OF USE FOR A MAJOR OUTER CAPSID PROTEIN (VP7) OF ROTAVIRUS SA-11

The present invention pertains in general to viral proteins and assays and vaccines employing them and, in particular, to proteins related to the rotavirus major outer capsid protein.

Human rotavirus has been shown in recent years to be the principal causative agent of diarrhea in infants and young children. Epidemological research suggests that rotaviruses are ubiquitous in nature and constitute a major health problem worldwide, particularly in underdeveloped countries where combined with the effects of malnutrition, they can cause chronic illness and death.

Rotaviruses are members of the virus family Reoviridae and are characterized by double-stranded RNA genomes arranged in 11 segments as identified by differences in their electrophoretic mobility. Kapikian et al., "Rotaviruses", Chapter 37 in Virology, pp. 863-906, Fields ed., Raven Press, New York, (1985). Ultrastructural analyses show that rotavirus gene segments are packaged in a core particle surrounded by inner capsid and outer capsid layers. Palmer et al., J. Gen. Virol., 35, 403-414 (1977); and Palmer et al., J. Gen. Virol., 62, 105-111 (1982); Bican et al., J. Virol., 43, 1113-1117 (1982). The proteins comprising the core, inner, and outer capsid are now well characterized, particularly for the simian rotavirus SA-11 and bovine rotavirus UK. Gene coding assignments exist for most of the proteins. Kapikian et al., supra.

Four human serotypes of rotavirus have been described. These are distinguished by inability of antisera prepared against virus of each serotype to neutralize virus infectivity of other serotypes in a plaque reduction assay. The prototype strains of these four serotypes are Type 1 (Wa), Type 2 (DS-1, S2), Type 3 (M, P) and Type 4 (St. Thomas), Wyatt et al., J. Clin. Microb., 18, 310-317 (1983). Virus isolates that may represent two additional human serotypes have also been described, Matsuno et al., J. Virol., 54, 623-624 (1985); Albert, Acta Paediatr. Scand., 74, 975-976 (1985); Clark et al., Amer. Soc. for Microb., 1986 Annual Meeting, Washington, DC, Abstract T34.

In addition to the four human serotypes, three serotypes have been described representing primarily porcine (Type 5), bovine (Type 6) and avian (Type 7) rotaviruses. Viruses of two of the human serotypes also cross-react with animal rotaviruses. Simian (SA-11, MMU18006) and canine (CU-1) rotaviruses cross-react with serotype 3 human viruses, and several strains of porcine rotavirus cross-react with human serotype 4. Kapikian et al., supra.

Genomic reassortant studies performed by coinfecting human rotaviruses not adapted for growth in tissue culture with animal rotaviruses have identified the principle gene product responsible for virus neutralization as the major outer capsid protein (VP7). VP7 has been shown to be coded for by gene 8 or 9 depending on serotype by reassortant studies and in vitro translation. Kapikian et al., supra.

VP7 is heterogeneous in size (36-38 kilodaltons) and is glycosylated with a high mannose carbohydrate. Estes et al., Virology, 122 8-14 (1982); Kouvelous et al., J. Gen. Virol., 65, 1211-1214 (1984). The absence of the carbohydrate moiety does not inhibit rotavirus infectivity or influence virus neutralization. Petrie et al., J. Virol., 46, 270-274 (1983); Sabara et al., J. Virol., 53 58-66 (1985).

The gene coding for VP7 (gene 8 or gene 9) has been cloned as a cDNA copy in E. coli and sequenced for human Type 1 (Wa), human Type 2 (Hu-5), simian Type 3 (SA-11), and bovine Type 6 (UK, NCDV) rotaviruses. Glass et al., Virology, 141, 292-298 (1985). Analysis of the inferred amino acid sequences reveals two tandem hydrophobic domains near the N-terminus of VP7 each preceded by a methionine codon (ATG). Translation studies in vitro suggest that both ATG codons are used during virus infection. Ericson et al., Virology, 127, 320-332 (1983); Chan et al., Virology, 151, 243-252 (1986).

Comparison of the inferred amino acid sequences of VP7 for the rotavirus types reveals six regions which are particularly variable in sequence. These "hypervariable" regions are thought to be likely candidates for epitopes which confer rotavirus type specificity. Glass et al, _supra_. Peptides have been made to these variable regions and their antigenicity evaluated. Gunn et al., _J. Virol._, _54_, 791−797 (1985). While some of the peptides were antigenic, none were capable of eliciting a neutralizing antibody response. These data suggest that conformational features of the VP7 protein are essential to affect virus neutralization.

Attempts have been made to purify VP7 from virus particles to use as a reagent for generating neutralizing immune sera. Subunit VP7 purified by isoelectric focusing was used to generate neutralizing mono-specific sera. Matsuno and Inouye, _Infec. and Immun._, _39_, 155−158 (1983). Similar studies using denatured VP7 or proteolytic fragments of VP7 have shown that conformational features on the protein are important for eliciting a neutralizing antibody response. Bastardo et al., _Infec. and Immun._, _34_, 641−647 (1981); Sabara et al., _J. Virol._, _56_, 1037−1040 (1985); Sabara et al., _J. Virol._, _53_, 58−66 (1985).

Attempts to express rotavirus VP7 in _E. coli_ as a source of antigen to raise neutralizing antibody have not been successful. Although truncations of the gene coding for VP7 have been expressed in E. coli using various vector constructions, stability of the polypeptides has been a problem, as well as low yields presumably due to toxicity in E. coli. Such polypeptides have been shown to be antigenic, but do not generate neutralizing antibodies to rotavirus, presumably due to incorrect protein folding.

Gene 9 of simian rotavirus SA-11 has been expressed in mammalian cells (COS-7) using SV40 vectors. A series of deletions was constructed which showed that both ATG codons proximal to the two hydrophobic domains were used in translation, but the VP7 protein products similar to native protein were not secreted. Poruchynsky et al., J. Cell Biol., 101, 2199-2209 (1985).

Baculovirus has been developed as a system for expression of foreign genes. Smith et al., Molec. Cell. Biol., 3, 2156-2165 (1983); Pennock et al., Molec. Cell. Biol., 4, 399-406 (1984). Some of these gene products have been found to be secreted into the tissue culture media, Smith et al., supra; Smith et al., Proc. Natl. Acad. Sci., 82, 8404-8408 (1985); while others have not. Miyamoto et al., Molec. Cell. Biol., 5, 2860-2865 (1985). In most cases, biological activity has been maintained. Smith et al., supra; Carbonell et al., J. Virol., 56, 153-160 (1985); Maeda et al., Nature, 315, 592-594 (1985). A baculovirus expression system has been used recently for expression for rotavirus SA-11 major inner capsid protein (VP6). Summers and Smith, Banbury Rep., 22, 319-339 (1985).

The present invention provides a recombinant subunit form of the major outer capsid protein (VP7) of rotavirus SA-11. The protein is produced by expression of rotavirus SA-11 gene 9 in a baculovirus expression system and enables the synthesis of a 34 kilodalton (34K) protein, a size consistent with that expected for the native protein. The protein is secreted from the insect cells into the culture medium during virus infection. The protein is antigenic and is recognized by neutralizing anti-rotavirus antiserum, and is useful as a diagnostic reagent or as an antigen for an active or passive vaccine.

An assay for detecting the presence of anti-rotavirus antibodies in a sample according to the present invention includes the step of exposing a sample containing antibody to the recombinant protein according to the present invention. The recombinant protein is bound to a support, reacts immunologically with antibody in the sample, and immobilizes the antibody in the sample onto the support. A second antibody, capable of specifically binding with the antibody in the sample and associated with a label (e.g., an enzyme label, a fluorescent label or other labels conventional in the art) is introduced into the test solution. The presence of label bound to the support indicates the presence of antibody against rotavirus in the sample.

An active vaccine according to the present invention includes an immunologically acceptable diluent, adjuvant or carrier and the recombinant protein according to the present invention.

A method of raising polyclonal antibodies according to the present invention includes the steps of immunizing an animal with an effective amount of the recombinant protein according to the present invention, and isolating anti-VP7 antibodies from serum, collostrum, milk, or other body fluid of the animal. The present invention also provides antibodies raised according to this method.

A method of producing monoclonal antibodies according to the present invention includes the steps of immunizing an animal against the recombinant protein according to the present invention, fusing antibody-producing cells from the animal to myeloma cells; isolating a cell line producing a monoclonal antibody against VP7 in a medium, and purifying monoclonal antibody against VP7 from the medium. The present invention also provides monoclonal antibodies produced by this method.

Figure 1 is a schematic depiction of the construction of a library of cDNA according to the present invention;

Figure 2 is a partial restriction map of a rotavirus SA-11 gene 9 cDNA according to the present invention;

Figure 3 is a comparison of the nucleic acid sequence for rotavirus SA-11 gene 9 according to the present invention with published sequences;

Figure 4 is a schematic illustration of the construction containing the gene coding for the recombinant protein for insertion into the baculovirus genome according to the present invention.

In accordance with the present invention, rotavirus SA-11 RNA gene segments were used as templates to synthesize DNA copies which were subsequently cloned into plasmid vectors in E. coli. A cloned DNA copy of SA-11 gene 9 was identified and verified by DNA sequence analysis to contain the entire protein coding sequence of the major outer capsid protein (VP7).

SA-11 gene 9 cloned DNA was expressed under control of the polyhedrin promoter to yield a subunit protein antigenically similar to the native protein, by infection of an insect cell line with recombinant baculovirus containing gene 9. The resulting expressed gene product has been shown to be antigenic by reacting with neutralizing polyclonal antisera derived against rotavirus particles.

The baculovirus-expressed SA-11 gene 9 protein product has utility as a diagnostic reagent to detect antibodies to rotavirus in clinical samples and as an immunogen for production of polyclonal or monoclonal antibodies to the rotavirus major outer capsid protein. Expression of SA-11 gene 9 in baculovirus also allows for modification of the antigenic determinants of the recombinant VP7 protein product by site specific mutagenesis of the cloned gene 9 DNA.

In the following examples, restriction endonucleases were supplied by New England Biolabs (NEB). DNA polymerase Klenow fragment, Poly(A) polymerase, and DNA ligase were purchased from Bethesda Research Labs (BRL) Gaithersburg, Maryland. Reverse transcriptase was supplied by Life Sciences. RNA ligase and terminal transferase were supplied by Pharmacia Molecular Biologicals (PMB). $^{32}$P-labeled reagents, including nick translation reagents, were purchased from New England Nuclear (NEN). $^{35}$S-labeled reagents, were purchased from Amersham. Agarose, acrylamide, and other electrophoresis reagents were supplied by Bio Rad. Growth media were obtained from Difco. X-gal was purchased from BRL. Media and buffers for growth of SA-11 rotavirus and Sf9 insect cells were obtained from Grand Island Biological Company (GIBCO).

E. coli strains JM 83, JM 103, and HB101 competent cells were obtained from PMB and BRL. Plasmids for cloning and expression (pUC9, pUC13) were purchased from PMB. M13 vectors (mp8, mp9) and primers for Sanger sequencing were purchased from PMB. Baculovirus E. coli shuttle vector (pAc611) was obtained from Max D. Summers, Texas A&M University.

Standard procedures were employed for: plasmid purification, transformation of E. coli, small scale plasmid preparation, analyses of DNA by agarose gel eletrophoresis, screening of recombinant colonies using X-gal, and colony hybridization, [Maniatis et al., Molecular Cloning - A Laboratory Manual, Cold Spring Harbor Laboratory, Cold Spring Harbor, New York (1982)]; and for analyses of proteins by SDS-acrylamide gel electrophoresis (PAGE) [Laemmli, Nature, 227, 680-685 (1970)]. Restriction enzymes (NEB), RNA ligase (PMB), poly(A) polymerase (BRL), terminal transferase (BRL), DNA polymerase Klenow fragment (BRL), and DNA ligase (BRL) were used according to the manufacturers' recommendations.

The following examples set forth the present invention in greater detail. Example 1 describes the synthesis of rotavirus SA-11 cDNAs and cloning thereof in E. coli. Example 2 details the identification and mapping of SA-11 gene 9 cloned DNA. In Example 3, the results of a Northern blot analysis of SA-11 gene 9 cloned DNA are presented. Example 4 describes a sequence analysis of SA-11 gene 9 cloned DNA. In Example 5, a description of the subcloning of SA-11 gene 9 DNA for expression in baculovirus is provided. Example 6 details insertion of SA-11 gene 9 into baculovirus by transfection, in an insect cell system. Example 7 provides a demonstration of the synthesis of an SA-11 gene 9 cloned DNA product (VP7) in a baculovirus-insect cell system and measurement of the apparent molecular weight of the product. Example 8 provides a demonstration of antigenicity of the VP7 as expressed in a baculovirus-insect cell system. Example 9 details the secretion into the insect tissue culture media of VP7 in a baculovirus-insect cell system. Example 10 provides an enzyme-linked immunosorbent assay according to the present invention. In Example 11, polyclonal antisera are produced according to the present invention and a vaccine according to the present invention is described. In Example 12, monoclonal antibodies are produced according to the present invention.

## Example 1

### Propagation and Purification of SA-11 Rotavirus

SA-11 rotavirus [ATCC No. VR899] was propagated by infection of confluent MA104 cells (MA. Bioproducts) in roller bottles as indicated by Estes et al., J. Virol., 31, 810-815 (1979), except that tryptose phosphate and gentamicin were omitted from the media, and phosphate buffered saline (PBS, available from GIBCO) was used as a wash buffer instead of Tris buffered saline (TBS). Virus was propagated in the absence of trypsin or fetal calf serum and harvested after two days growth at 37$^{\circ}$C. SA-11 stock virus was activated prior to infection by treatment with a 1/100 volume 10X tissue culture grade trypsin (GIBCO) for one hour at room temperature. Virus was purified by freeze-thawing infected cells two times followed by centrifugation for 20 minutes at 5000g and at 4$^{\circ}$C to remove cellular debris. Eight percent (w/v) polyethylene glycol M.W. 6000 (PEG) was added to the clarified supernatant and allowed to stand overnight at 4$^{\circ}$C. The media was then centrifuged 20 minutes at 5000g (4$^{\circ}$C) to pellet PEG-precipitated SA-11 virus. The pellet containing virus was solubilized in a small volume of 50mM Tris-HCl (pH 7.5) buffer and applied to a step gradient consisting of 1.4 g/ml CsCl, 1.3 g/ml CsCl, and 45% (w/v) sucrose in Tris buffer (20$^{\circ}$C). Samples were centrifuged in a swinging bucket rotor for 3-6 hours at 100,000g (20$^{\circ}$C). Virus particles, seen as a sharp bluish band migrating between 1.3-1.4 g/ml CsCl, were harvested and dialyzed overnight against 50 mM Tris-HCl (pH 7.5). Dialyzed virus served as purified source material for isolation of genomic double-stranded RNA (dsRNA) or synthesis of virally coded messenger RNA (mRNA).

Isolation of SA-11 dsRNA and $^{32}$P-end Labeling

Double-stranded RNA was extracted from CsCl-purified SA-11 virus by addition of an equal volume of Tris-HCl (pH 7.5) buffer-saturated phenol followed by vortexing (phenol extraction). Double-stranded RNA contained in the aqueous phase was concentrated by precipitation with 0.2M sodium acetate (Na Ac) and two volumes of ethanol then stored at $-20^{\circ}$C until use.

SA-11 dsRNA was end-labeled to serve as a size marker when resolving polyadenylated dsRNA by agarose gel electrophoresis or for Northern blot analysis of SA-11 dsRNA segments resolved by acrylamide gel electrophoresis.

Double-stranded RNA was labeled by addition of $^{32}$P-Cp (cytosine 5', 3'-diphosphate) to the 3'-termini of the RNA strands using RNA ligase as described by the manufacturer (PMB).

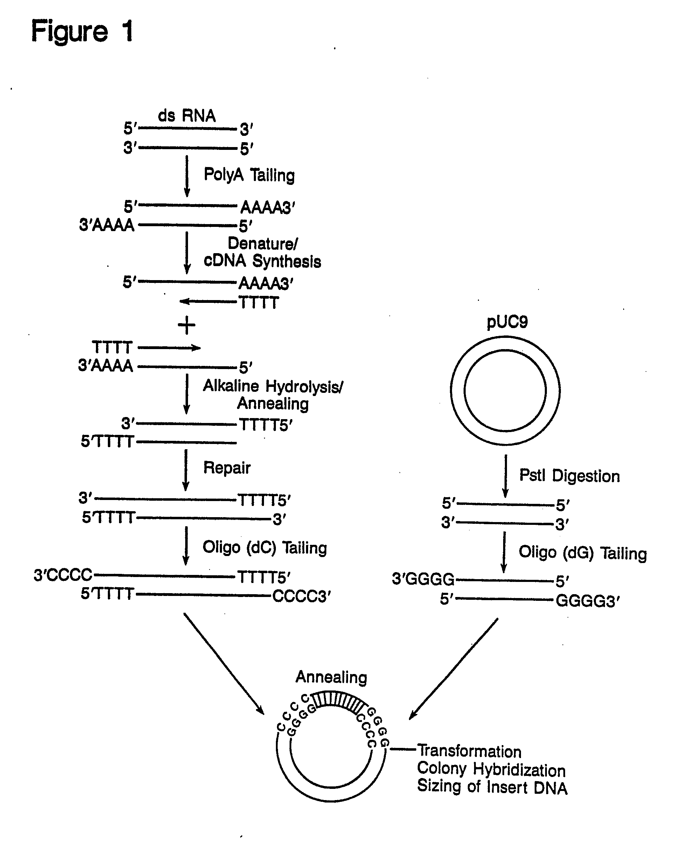

As shown in Fig. 1, a mixture of 11 segments of SA-11 double-stranded RNA (dsRNA) was isolated from SA-11 virus particles.

Polyadenylation of SA-11 dsRNA

Adenosine residues were added to the 3'-termini of SA-11 dsRNA (poly(A)-tailed dsRNA) to provide a template binding site for cDNA synthesis using reverse transcriptase (Fig. 1). Standard methods were used (i.e., as recommended by BRL) except that 2.5 U/ul of RNasin (Promega Biotech) was added as a nuclease inhibitor. Five to ten micrograms (ug) of dsRNA were polyadenylated in a two minute reaction at 37°C resulting in the addition of 20-40 adenosine residues. $^3$H-ATP was usually included at 10,000 Ci/mol in the reactions to measure RNA concentrations. Occasionally, (alpha-$^{32}$P) ATP (10,000 Ci/mol) was added for size analysis of polyadenylated (poly(A)) dsRNA by electrophoresis on a one percent agarose gel. $^3$H-labeled poly(A) dsRNA was stored in 70% ethanol (-20°C) until used for cDNA synthesis (Fig. 1).

First Strand cDNA Synthesis of SA-11 dsRNA

Poly(A)-tailed SA-11 dsRNA (1-2ug) was lyophilized then strand separated by resuspending in 5ul of 90 percent DMSO (Aldrich-Spectrophotometric Grade) and incubating 30 min. at 37°C. Strand separated RNA was added last to complete a 100ul reaction mixture containing: 50mM Tris-HCl (pH 7.5), 10mM magnesium acetate, 10mM dithiothreitol (DTT), 2mM dATP, 2mM dGTP, 2mM dTTP, 0.2mM (alpha-$^{32}$P)dCTP (10,000 Ci/mol), 100ug/ml oligodT$_{(12-18)}$ (PMB) (used as a primer), 0.6uU/ 1 RNasin, and 0.4 U/ul reverse transcriptase (Life Sciences). The reactants were incubated for 1 hour at 37°C, then synthesis was stopped by addition of an equal volume of Tris-buffer saturated phenol. The aqueous phase was adjusted to 20mM EDTA, and 0.2 N NaOH and incubated overnight at 37°C to remove template RNA, then neutralized with an equal volume of 0.2 N HCl and applied to a Sephadex$^R$ G100 column (Pharmacia) to remove salts and unreacted nucleotides. Fractions containing $^{32}$P-labeled SA-11 cDNA were collected and the DNA was concentrated by precipitation with 0.2M Na Ac and 2 volumes of ethanol (-20°C). Typically, 0.1-0.2 ug cDNA were synthesized per microgram of template dsRNA. The integrity of SA-11 cDNA was evaluated by electrophoresis on a one percent alkaline agarose gel containing 30mM NaOH, 2mM EDTA to remove secondary structures.

## Annealing and Second Strand Synthesis of SA-11 cDNA

SA-11 cDNA was resuspended in 20 microliters of 50mM Tris-HCl (pH 7.5) and 0.1 M NaCl, then heated for 5 min. 65°C followed by 1 hour at 55°C; and slow cooled to promote annealing of complementary cDNA strands. Annealed cDNA was then precipitated by addition of two volumes of ethanol (-20°C).

Annealed cDNA was treated again with reverse transcriptase to render any remaining single-stranded ends double-stranded (Fig. 1). 0.1-0.2ug annealed SA-11 cDNA was added to a reaction mix identical to that used for first strand synthesis without dsRNA. The reaction was carried out for 30 minutes at 42°C then stopped by addition of an equal volume of Tris buffer-saturated phenol. The aqueous phase, after extraction with phenol, was precipitated with 0.2M Na Ac and 2 volumes of ethanol (-20$^{O}$C).

Size Selection of SA-11 Double-Stranded cDNA

$^{32}$P-labeled SA-11 double-stranded cDNA was resolved by electrophoresis on a 1 percent agarose gel containing TBE buffer (89mM Tris-base, 89mM boric acid, and 2.5mM EDTA) using standard methods [Maniatis et al., Molecular Cloning - A Laboratory Manual, Cold Spring Harbor Laboratory, Cold Spring Harbor, New York, (1982)]. DNA digested with endonuclease HindIII and X DNA digested with endonuclease HaeIII were applied as size markers. The gel was stained with 0.01 percent ethidium bromide (EtBr) to visualize the size markers. $^{32}$P-labeled gene 9 DNA, visualized by autoradiography of the gel on X-ray film (Kodak XAR-5), and migrating in a size range of 1000-1100 base pairs as judged by size markers, was excised from the gel. The gel slice was placed in a dialysis bag containing 0.1X TBE buffer, and the DNA was electroeluted for 4 hours at 100 volts in 0.1X TBE. The eluted DNA was concentrated and purified using an Elutip$^{TM}$-d column (Schleicher and Schuell) following the manufacturers' specifications. Purified, size-selected SA-11 cDNA was stored in ethanol (-20$^{O}$C) until further use.

## Terminal Transferase Tailing of SA-11 cDNA

Deoxycytidine nucleotides (dC) were added to the 3' termini of SA-11 double-stranded cDNA (C-tailing) using terminal transferase (PMB) as shown in Fig. 1. 0.01-0.1ug cDNA was C-tailed according to the manufacturers' recommendations (BRL) in 20ul reactions using 10 uM $^3$H-dCTP (100,000 Ci/mol). Reactions were allowed to proceed for 3 minutes at 37$^o$C resulting in addition of 10-20 dC residues per DNA 3'-end. Reactions were stopped with an equal volume of Tris buffer-saturated phenol. C-tailed cDNA recovered in the aqueous phase after phenol extraction was precipitated with 0.2 M Na Ac and 2 volumes of ethanol (-20$^o$C).

## Annealing of C-Tailed SA-11 cDNA with G-Tailed Plasmid DNA and Cloning in E. coli

0.01-0.1 ug C-tailed SA-11 cDNA was suspended in 50mM Tris (pH 7.5), 0.1M NaCl, and an equal number of picomoles of pUC9, cut with PstI and G-tailed (PMB), was allowed to anneal by heating 5 minutes at 65$^o$C then for 1 hour at 55$^o$C before being allowed to slow cool (Fig. 1). The annealed cDNA-plasmid was precipitated with 2 volumes of ethanol (-20$^o$C).

100ul of E. coli JM 83 cells were made competent using CaCl$_2$ according to standard procedures [Maniatis et al., Molecular Cloning - A Laboratory Manual, Cold Spring Harbor Laboratory, Cold Spring Harbor, New York (1982)] and were added to lyophilized SA-11 cDNA-pUC9 DNA ($0^{O}C$). After 1 hour on ice, the cells were heat-shocked for 2 minutes at $42^{O}C$, incubated in 1 ml of LB (Luria Broth) media for 1 hour at $37^{O}C$ and applied to LB agar plates containing ampicillin (Amp) and the color indicator X-gal. Colonies containing pUC9 metabolize X-gal resulting in a blue phenotype. [Maniatis et al., Molecular Cloning - A Laboratory Manual, Cold Spring Harbor Laboratory, Cold Spring Harbor, New York (1982)]. When the lacZ' sequence of pUC9 is interrupted by the presence of an insert, a white colony results. White colonies were replated and assayed further by colony hybridization.

## Example 2

Clones were screened initially by the presence of white colonies on agar plates containing X-gal indicating plasmids which contained SA-11 cDNA sequences. The clones were re-screened by colony hybridization using a radiolabeled gene 7, 8, 9 mRNA mixture as a probe. Colonies which hybridized positively to gene 7, 8, 9 mRNA were further characterized by sizing of the insert DNAs and mapping with restriction endonucleases.

## Synthesis of SA-11 mRNA Probe and Purification of Gene 7, 8, 9 mRNA

$^{32}$P-labeled SA-11 mRNA was synthesized for use as a probe from CsCl-purified SA-11 virus in a 100ul reaction containing: 60mM Tris-HCl (pH 8.3), 6mM Mg(Ac)$_2$, 100mM Na Ac, 2mM GTP, 0.1mM ATP (10,000 Ci/mol), 0.1mM CTP (10,000 Ci/mol), 0.1mM UTP (10,000 Ci/mol), 1.0mM S-adenosyl methionine (SAM), 0.6U/ul RNasin, and 10ug of SA-11 virus. Reactions were carried out for 4 hours at 37$^O$C then stopped by extraction with an equal volume of Tris buffer-saturated phenol. $^{32}$P-labeled mRNA was applied to a Sephadex® G100 column to remove unreacted nucleotides. Labeled fractions containing mRNA were pooled and concentrated by precipitation with 0.2M Na Ac and 2 volumes of ethanol (-20$^O$C). $^{32}$P-labeled SA-11 mRNA species were resolved by electrophoresis on a 1.5 percent agarose gel containing E buffer (18mM Na$_2$HPO$_4$, 2mM NaH$_2$PO$_4$) and 6 percent formaldehyde as a denaturing agent. Messenger RNA samples were denatured before loading onto the gel by heating for 5 min. at 65$^O$C in E buffer, 6 percent formaldehyde, and 50 percent formamide, then quick cooling on ice. λDNA, digested with HindIII, and φX DNA, digested with HaeIII, were applied to the gel as size markers. The gel was stained with 0.01 percent ethidium bromide (EtBr) to visualize the DNA markers. The mRNA species migrating at a mobility expected for gene 7, 8, 9 mRNA (1000-1100 base pairs) were excised from the gel, the gel slice was inserted into a dialysis bag containing 0.1X TBE buffer, and the mRNAs were electroeluted for 4 hours at 100 volts in 0.1X TBE. The eluted mRNA was concentrated and purified using an Elutip$^{TM}$-d (Schleicher and Schuell) following the specifications of the manufacturer. Gene 7, 8, 9 mRNA was stored in ethanol (-20$^{O}$C) until use as a probe for colony hybridization.

## Colony Hybridization And Small Scale Plasmid Screening Of SA-11 Gene 9 Cloned DNA

Colonies derived from cloning SA-11 cDNA into E. coli strain JM 83 were screened for the presence of gene 9-specific DNA sequences by colony hybridization using $^{32}$P-labeled SA-11 gene 7, 8, 9 mRNA as a probe. Colony hybridization was carried out by the method of Grunstein and Hogness using standard protocols. Maniatis et al., Molecular Cloning - A Laboratory Manual, Cold Spring Harbor Laboratory, Cold Spring Harbor, New York (1982). Colonies which were identified as hybridizing to gene 7, 8, 9 mRNA by autoradiography of X-ray film (Kodak XAR-5) were amplified by incubation overnight in LB media.

E. coli cells containing gene 7, 8, 9 DNA sequences were pelleted and lysed following the standard procedures of Birnboim and Doly, [Maniatis et al., Molecular Cloning - A

*Laboratory Manual*, Cold Spring Harbor Laboratory, Cold Spring Harbor, New York (1982)]. Plasmid DNAs recovered from the cell supernatant were digested with PstI endonuclease to excise insert DNAs. The PstI-cut plasmid DNAs were resolved by electrophoresis on 1 percent agarose gels in TBE buffer along with λDNA cut with HindIII and φX HaeIII-cut DNA as size markers. Plasmids containing apparently full-size inserts of gene 7, 8, 9 DNA were mapped further with restriction endonucleases. The size of the excised inserts was determined by mobility on agarose gel electrophoresis.

Restriction Endonuclease Mapping Of SA-11 Gene 9 Cloned DNA

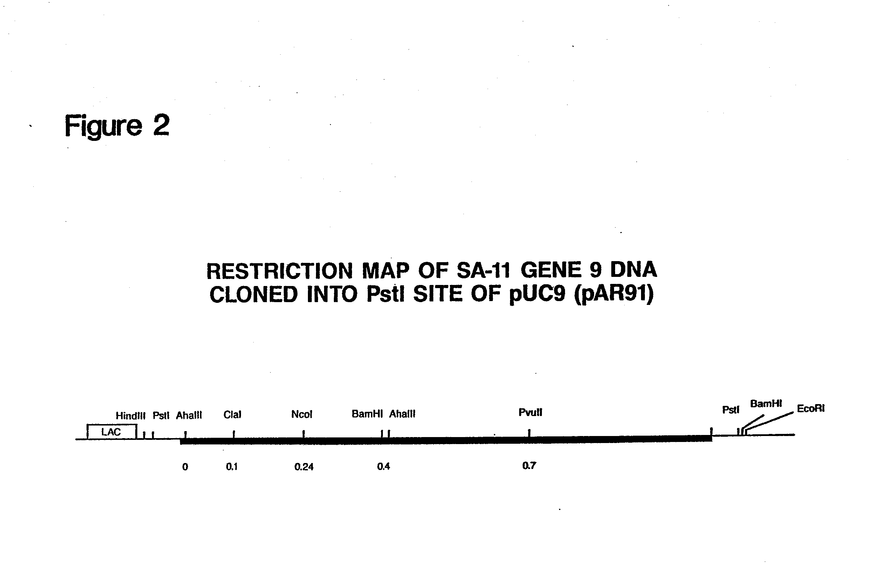

Gene 7, 8, 9 DNAs contained in plasmid pUC9 were mapped with restriction endonucleases AhaIII, ClaI, NcoI, BamHI, PvuII, and PstI to better estimate the size of the insert DNA and to compare with published restriction sites for SA-11 gene 9 reported by Both et al., *Proc. Natl. Acad. Sci.*, 80, 3091-3095 (1983); and Arias et al., *J. Virol.*, 50, 657-661 (1984). The resulting partial restriction map as illustrated in Fig. 2 confirms the presence of a full-size insert of SA-11 gene 9 cDNA. The orientation of gene 9 DNA in pUC9 (pAR91) was determined by digestion with BamHI which is contained within the universal cloning site of pUC9 and flanks the insert site of gene 9 DNA as well as within the gene 9 sequence. Analysis of the restriction digests by agarose gel electrophoresis showed that gene 9 was inserted in the forward orientation with respect to the lac promoter.

## Example 3

To confirm that the DNA inserted in pAR91 was SA-11 gene 9, a radiolabeled probe was made from gel purified insert DNA and hybridized to a mixture of SA-11 dsRNAs resolved by electrophoresis on an acrylamide gel and blotted onto a Genescreen[TM] membrane (NEN).

## Northern Blot Hybridization of SA-11 Gene 9 DNA

SA-11 gene 9 cloned DNA was excised from pAR91 with PstI endonuclease then purified by electrophoresis on a 1 percent agarose gel in TBE buffer followed by excision of the gene 9 fragment, electroelution, and recovery by Elutip[TM]-d procedures as described above for purification of SA-11 cDNA. Purified gene 9 DNA was $^{32}$P-labeled in a nick translation reaction using (alpha-$^{32}$P)dCTP according to the manufacturers' protocol (NEN). Nick translated gene 9 DNA served as a $^{32}$P-labeled probe for hybridization to gene 9 by a Northern blot technique.

Unlabelled SA-11 dsRNA and $^{32}$P-Cp labelled dsRNA used as a size marker were resolved by electrophoresis on a 5 percent acrylamide gel in TBE buffer. The resolved RNAs were denatured by treatment with 0.1 M NaOH for 15 min. at room temperature followed by washing with sodium phosphate buffer (pH 5.5) using the method of Street et al., _J. Virol._, _43_, 369-378 (1982). The denatured RNAs were then vacuum blotted onto Genescreen[TM] membrane (NEN) following the procedure of Peferoen et al., _FEBS Letters_, _145_, 369-372 (1982), using 20X SSC, 0.025 M $Na_2HPO_4/NaH_2PO_4$ (pH 6.5) as the blotting buffer.

Blotted RNAs were hybridized with gene 9 $^{32}$P-labeled nick translated probe using standard hybridization protocols with 50 percent formamide. Maniatis et al., _Molecular Cloning - A Laboratory Manual_, Cold Spring Harbor Laboratory, Cold Spring Harbor, New York (1982). Hybridization of gene 9 probe to specific SA-11 dsRNA sequences was determined by autoradiography using X-ray film (Kodak XAR-5).

Analysis of the Northern blot showed that the cloned DNA probe hybridized specifically to genome segment 9 confirming that the insert is a cloned DNA copy of gene 9 dsRNA.


### Example 4

The cloned DNA of SA-11 gene 9 was sequenced according to the method of Sanger [Sanger et al., _Proc. Natl. Acad. Sci._, _74_, 5463-5467 (1977)], using M13 cloning vectors and primers as follows.

Gene 9 was excised with PstI and purified by agarose gel electrophoresis using low melting temperature agarose (Sea Plaque) then applied to a NACS- Prepac™ column (BRL), washed, and eluted. The purified PstI fragment was cut with restriction endonucleases BamHI, ClaI, and PvuII.

The resulting fragments were ligated to M13 cloning vectors [mp8 and mp9-(PMB)] containing complementary sites as follows: a 100 base pair (bp) PstI, ClaI fragment ligated to mp9 cut with PstI, AccI; a 300bp ClaI, BamHI fragment ligated to mp8 and mp9 cut with AccI, BamHI; a 310bp BamHI, PvuII fragment ligated to mp8 and mp9 cut with BamHI, SmaI; a 450bp PvuII, PstI fragment ligated to mp8 and mp9 cut with SmaI, PstI; a 660bp BamHI, PstI fragment ligated to mp8 and mp9 cut with BamHI, PstI. All ligated fragments were cloned into E. coli JM 103 strain. Phage plaques were picked and amplified to obtain single-stranded DNA templates for sequencing using standard plasmid purification methodologies. Maniatis et al., supra.

Templates were sequenced using M13 oligonucleotide primers according to standard Sanger methodologies specified by the manufacturer (PMB).

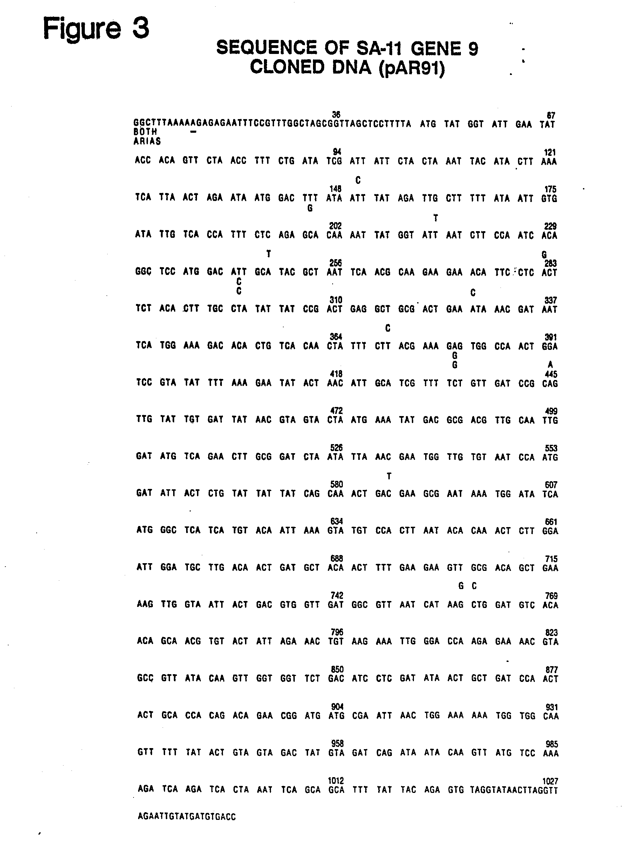

Analysis of the DNA sequence of the SA-11 cloned DNA, as provided in Fig. 3, showed that the entire gene 9 sequence was present. Comparison of the sequence of the SA-11 cloned DNA to other published sequences reported by Both et al., Proc. Natl. Acad. Sci., 80, 3091-3095 (1983); and Arias et al., J. Virol., 50, 657-661 (1984), showed 12 base pair differences when compared to the sequence of Arias et al., and 4 differences when compared to Both et al. The inferred amino acid sequence derived from the DNA sequence has eight differences compared to that of Arias et al., and three differences compared to Both et al. Of the amino acids unique to our clone, only two changes at position 65 (I--T) and position 110 (E--G) are not conserved.

## Example 5

## Subcloning of SA-11 Gene 9 DNA for Baculovirus Expression

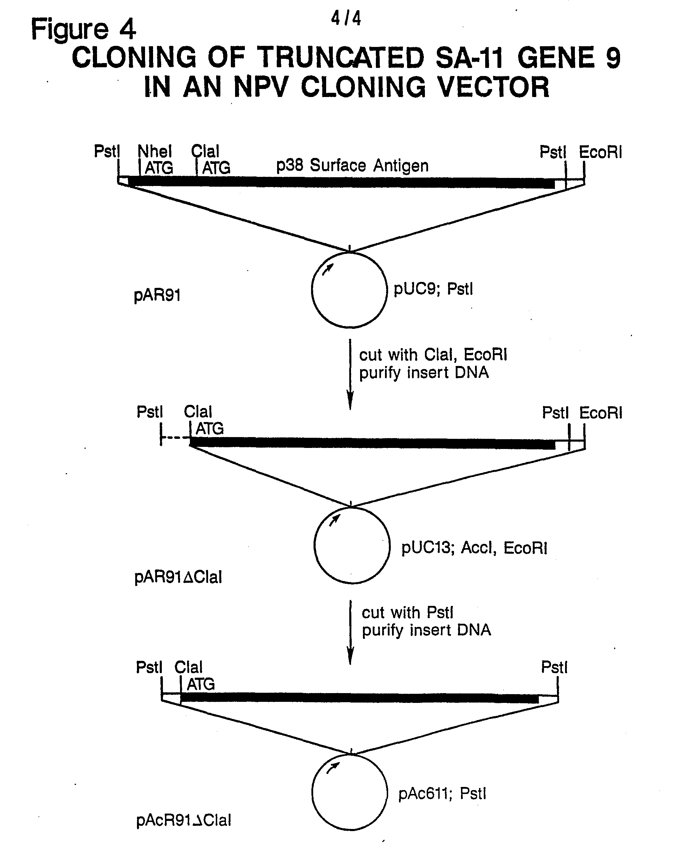

As shown in Fig. 4, gene 9 DNA was excised from pAR91 using restriction nucleases ClaI and EcoRI, which allowed for removal of 5'-terminal homopolymeric dG flanking DNA as well as the first initiator methionine and first hydrophobic domain. The insert DNA was purified by gel electrophoresis, electro-elution, and Elutip$^{TM}$-d methodologies described above. The insert DNA was joined to pUC13 using DNA ligase according to the suppliers specifications (BRL). The plasmid pUC13 had been digested with AccI and EcoRI prior to ligation. The ligated DNA was cloned into E. coli strain HB101 by standard methodologies using competent cells that were commercially prepared (BRL).

Maniatis et al., <u>Molecular Cloning - A Laboratory Manual</u>, Cold Spring Harbor Laboratory, Cold Spring Harbor, New York (1982). The resulting construction (pAR91ΔClaI) placed gene 9 in the forward orientation with respect to the <u>lac</u> promoter and allowed for excision of the insert by endonuclease <u>Pst</u>I.

Gene 9 was then excised from pAR91ΔClaI with <u>Pst</u>I and gel purified as described above. The purified insert was ligated to an <u>E. coli</u> baculovirus shuttle vector (pAc611) previously digested with <u>Pst</u>I and phosphatased with calf intestinal phosphatase (Boehringer) following the manufacturer's recommendations. The ligated DNA was cloned into <u>E. coli</u> strain HB101 using competent cells (BRL) by standard methodologies resulting in construction of pAcR91ΔClaI. Maniatis et al., <u>supra</u>.

The resulting protein contains the amino acid sequence shown in Table 1. The internal methionine codon at amino acid position 30 of the complete gene 9 sequence acts as the initiation codon in the baculovirus expression system due to its position as the 5'-proximal ATG in the insert sequence. Kozak, <u>Nuc. Acids Res.</u>, <u>12</u>, 857-872 (1984). As previously stated in the Background of the Invention, this codon is believed to function <u>in vivo</u> during viral synthesis in mammalian cells. It, therefore, represents an authentic start site for translation of VP7.

## TABLE 1

```
         10          20          30          40          50          60
  MDFIIYRLLF  IIVILSPFLR  AQNYGINLPI  TGSMDIAYAN  STQEETFLTS  TLCLYYPTEA

         70          80          90         100         110         120
  ATEINDNSWK  DTLSQLFLTK  EWPTGSVYFK  EYTNIASFSV  DPQLYCDYNV  VLMKYDATLQ

        130         140         150         160         170         180
  LDMSELADLI  LNEWLCNPMD  ITLYYYQQTD  EANKWISMGS  SCTIKVCPLN  TQTLGIGCLT

        190         200         210         220         230         240
  TDATTFEEVA  TAEKLVITDV  VDGVNHKLDV  TTATCTIRNC  KKLGPRENVA  VIQVGGSDIL

        250         260         270         280         290
  DITADPTTAP  QTERMMRINW  KKWWQVFYTV  VDYVDQIIQV  MSKRSRSLNS  AAFYYRV
```

## Example 6

## Transformation and Screening of Recombinant Baculovirus-Gene 9 Plaques

Baculovirus _Autographa_ _californica_ (AcNPV) was propagated in an insect cell line (Sf9) of the fall armyworm _Spodoptera_ _frugiperda._ Maintenance of insect cells and infection with AcNPV virus was carried out as described by Summers and Smith, _A Manual of Methods for Baculovirus Vectors and Insect Cell Culture Procedures_, available upon request from Texas A&M University, College Station, Texas (1986). Briefly, Sf9 cells were grown in Graces medium (GIBCO) containing lactalbumin hydrolysate (DIFCO) and yeastolate (DIFCO) (TNM-FH medium) supplemented with 10% fetal bovine serum (Hazelton) (complete medium). Cells were plated in 75 $cm^2$ flasks (Corning), maintained at $27^O$C without $CO_2$ gas, and split by shaking cells loose every two days. Virus was grown by allowing a virus stock to adsorb to the cells 1 hour, $27^O$C, followed by adding fresh medium and allowing the infection to proceed for 2-3 days. Virus infection was characterized by the appearance of polyhedral inclusion bodies (polyhedra). AcNPV was used as a source for viral DNA in transfections.

AcNPV virus was purified as indicated except that the sucrose gradient purification step was omitted. Summers and Smith, supra. Virus infected cells were pelleted to remove debris, then the supernatant fraction was centrifuged at high speed (30 minutes, 100,000g) and the viral pellet was recovered. Viral DNA was isolated by treatment of the virus with proteinase K in buffer containing 10% sarcosyl followed by extraction with phenol/chloroform/isoamyl alcohol (25:24:1). The DNA was recovered as an ethanol precipitate.

AcNPV viral DNA and E. coli-Baculovirus shuttle vectors (e.g., pAc611, pAcR91 ClaI) were transferred into insect cells by the transfection procedure outlined under Method II except that sonicated salmon sperm DNA replaced calf thymus DNA.

Summers and Smith, supra. AcNPV viral DNA and recombinant plasmid DNA were mixed with a HEPES-phosphate buffer. DNA was precipitated by addition of $CaCl_2$. DNA precipitate was added to insect cells covered by Graces medium. Viral infection could be observed by the appearance of polyhedra during a 4-6 day incubation.

Virus infection was quantitated using a viral plaque assay as indicated by Summers and Smith, supra, except that insect cells were allowed to attach to 60mm plates (Nunc) in complete medium prior to infection. Briefly, Sf9 cells attached to 60mm plates were overlayed with serial dilutions of AcNPV virus resulting from DNA transfection or viral infection of insect cells. After 1 hour, $27^{O}C$, cells were covered by an overlay of 1.5% low melting point agarose (Sea Plaque) containing complete medium. After 4-6 days, plaques could be visualized which were characterized by the appearance of concentrations of polyhedra. Incubations were allowed to take place in a humidified plastic box.

Viral plaques containing recombinant SA-11 gene 9 sequences were discerned by hybridization of plaque DNA with $^{32}P$-labeled nick translated probe derived from purified gene 9 DNA cut with AccI and ClaI endonucleases. Summers and Smith, supra. 60mm plates containing viral plaques overlaid with agarose were allowed to dry 2-4 hours. The agarose layers were removed and stored at 4°C. Nitrocellulose filter discs were placed in the dishes, wetted, and pressed against the cells. The filter discs were removed and treated essentially as described by Grunstein and Hogness for bacterial colony hybridization. Maniatis, supra. Gene 9 nick translated probe was made following the manufacturers' recommendations (NEN). Hybridized plaques were identified by autoradiography using X-ray film (Kodak XAR-5). Plaques containing gene 9 sequences were recovered by aligning autoradiograms of nitrocellulose filters, marked for position of recombinant AcNPV virus-gene 9 plaques, to agarose overlays using dye markers, then picking plaques from the agarose and suspending in complete media. Further rounds of plaque purification were done following the same methodology, except that agarose plugs were inspected visually and plaques were picked from areas seemingly lacking in polyhedra, for further study. Recombinant plaques were generally plaque purified two times.

To verify that plaque-purified recombinant viruses were free of contamination from wild type AcNPV virus, recombinant virus was seeded onto insect cells grown in 96 well plates and examined visually for the appearance of polyhedra after 2-3 days. Wells containing cells which lacked polyhedra but showed cytopathology were scored as plaque-purified occlusion minus phenotype (occ⁻). Media was removed from the wells and stored

at $4^{O}$C as innoculum. Cells in each well were lysed using NaOH followed by $NH_4$ Acetate and solutions were applied to a dot blot matrix (Schleicher and Schuell) then hybridized as before. Viral plaques which scored as occ⁻ and hybridized to SA-11 gene 9 probe were propagated for further study. Virus innocula from 96 well plates were used to amplify viral plaques by infecting Sf9 cells sequentially in 24 well plates, 6 well plates and 25 $cm^2$ flasks (Corning). Infected media from 25 $cm^2$ flasks provided virus for subsequent analysis.

## Example 7

## Identification of Rotavirus SA-11 Major Outer Capsid Protein (VP7) Produced in a Baculovirus Expression System

A plaque purified virus isolate (B11.3) which was scored as occ⁻ and hybridized to gene 9 probe, was selected for further characterization. Sf9 cells ($3 \times 10^6$) in 25 $cm^2$ flasks were infected at multiplicities of infection (moi) of 0.01, 0.1, 1.0 and 10 plaque forming units (PFU) per cell. Infections were allowed to proceed 40 hours, $27^O$C. After 40 hours infection, 1.0 and 10 PFU/cell showed the most cytopathic effect (CPE), but no polyhedra were apparent.

Media were removed and cells were washed in Graces media without methionine. $^{35}$S-labeled methionine (50 uCi/ml) was added to 1.5ml methionine-free Graces media and applied to the infected cells 4 hours, 27°C. The cells were then removed from the flasks and centrifuged for 5 minutes at 1000g. Media was stored at -20°C for further analysis.

Cells were further treated by washing in PBS buffer then resuspending the cell pellets in 100ul RIPA buffer per flask (150mM NaCl, 10mM Tris-HCl, pH. 7.2, 1% Na deoxycholate, 1% Triton X-100, 0.1 SDS, 1% Aprotinin) and put on ice 15 minutes. Nuclei were removed by centrifuging for 5 minutes, 10,000g and stored at -20°C. The remaining cytoplasmic fraction is referred to as cytosol.

$^{35}$S-labeled cytosols from uninfected Sf9 cells, AcNPV infected cells, and B11.3 infected cells were analyzed for protein products by electrophoresis using a 10% SDS-polyacrylamide gel (PAGE) with a 4% stacking gel according to methods described by Laemmli, Nature, 227, 680-685 (1970). Exposure of radiolabeled proteins was enhanced by treating the gel with ENlightning™ (NEN) and drying prior to placing under X-ray film (Kodak XAR-5) with a QuantaIII™ intensifying screen (Dupont) at -70°C.

Resolution of the $^{35}$S-labeled proteins by gel electrophoresis showed that a 34,000 kilodalton (34K) protein was synthesized in B11.3 infected cells which was not present in uninfected cells and had a similar mobility to polyhedrin protein synthesized in AcNPV infected cells. This protein appeared to be present in the cytosol in amounts similar to polyhedrin protein in AcNPV infected cells.

Example 8

Immunoprecipitation of VP7 from Recombinant Baculovirus-gene 9
Infected Insect Cells

$^{35}$S-labeled cytosols from uninfected cells, AcNPV
infected cells, and B11.3 infected cells, previously resolved by
PAGE, were examined for the ability of the labeled proteins to
react with neutralizing antiserum to SA-11 rotavirus (obtained
from M.K. Estes, Baylor University, Houston, Texas) in an
immunoprecipitation assay.  Labeled cytosols were reacted with
either no serum, normal guinea pig serum, or guinea pig
antiserum to SA-11 rotavirus previously shown to have a 1/50,000
neutralizing titer (M.K. Estes), following procedures according
to Mason et al., Virology, 33, 1111-1121 (1980).
Immune-complexed proteins were bound to Protein A
Sepharose®-CL4B (Pharmacia) and unbound proteins were removed
as shown by Mason et al., supra.  Protein A-precipitated
proteins were resolved by electrophoresis on a 10%
polyacrylamide gel with a 4% stacking gel according to the
procedures of Laemmli et al., supra.

The results clearly show that $^{35}$S-labeled proteins
from uninfected or AcNPV infected cells were not reactive to the
antisera tested by immunoprecipitation.  A unique 34K protein
from B11.3 cytosols was found to be reactive to guinea pig
anti-rotavirus serum, but not normal guinea pig serum, or in the
absence of serum.  These results show for the first time that a subunit protein similar to the major outer capsid protein (VP7) of a rotavirus strain (SA-11) can be synthesized as a subunit protein in the baculovirus expression system yielding an antigenic product.

## Example 9

### Secretion of VP7 into the Culture Medium during Expression in the Baculovirus Expression System

Tissue culture media from AcNPV infected and B11.3 infected cells, harvested after 4 hours labeling with $^{35}$S-methionine, were examined for the presence of labeled proteins. Analysis of the media by electrophoresis on a 10% polyacrylamide gel with a 4% stacking gel (Laemmli, supra) revealed a unique protein of 34K to be secreted from B11.3 but not AcNPV infected cells. Only two other protein species, found in both AcNPV and B11.3 infected cells, were present in significant amounts. These data are the first demonstration of the production of a subunit similar to the native rotavirus major outer capsid protein (VP7) as a secretable product in any expression system. Secretion of the VP7 product allows for greater ease of recovery of the protein in highly purified form, particularly if the insect cells are maintained in serum-free medium during virus infection.

## Example 10

An enzyme-linked immunosorbent assay (ELISA) was developed for detecting the presence of anti-rotavirus antibodies.  In the assay, tissue culture cytosols were derived from uninfected, AcNPV infected, and Bll.3 infected cells as described in Example 7 except that unlabeled methionine was present in the culture medium.  The cytosols were applied to a 96 well plate at 1/10, 1/50 and 1/100 dilutions in PBS buffer and allowed to stand overnight at 4$^O$C.  Unadsorbed cytosol was removed by washing 5 times with PBS.  2% BSA in PBS was added as a blocking reagent, incubating 1 hour at 37$^O$C, and the excess BSA was removed by washing 5 times with PBS.  Next, first antibody (guinea pig anti-SA-11 rotavirus) was added at a 1/2000 dilution and incubated 1 hour at 37$^O$C.  The wells were washed 5 times with PBS and a second antibody was added (goat anti-guinea pig) conjugated to horse radish peroxidase (HRPD) at a 1/2000 dilution for 1 hour at 37$^O$C.  After 5 washes with PBS, a color reagent was added consisting of o-Phenylenediamine .2HCl (OPD) in a citrate-phosphate buffer diluent containing 0.2% hydrogen peroxide.  The color reaction was allowed to develop 15 minutes at room temperature.  Positive antigen-antibody reactions appeared as an orange color in a plate well. The reactions were stopped by the addition of 1N $H_2SO_4$. Color intensities were quantitated by spectroscopy on an Immunoreader NJ-2000 spectrophotometer (Intermed).

Table 2 shows the detection of the major outer capsid protein (VP7) expressed in a baculovirus-insect cell system by ELISA. Expressed gene products of AcNPV gave similar background numbers as observed with uninfected cells, comparing reactivities to neutralizing antiserum against SA-11 rotavirus. By contrast, B11.3 which expresses recombinant VP7 was observed to give a reproducibly positive signal by ELISA.

### TABLE 2

| Dilution of Cell Lysate | Uninfected | NPV | B11.3 |
|---|---|---|---|
| 1/10 | 0.162 | 0.100 | 0.583 |
| 1/50 | 0.266 | 0.330 | 0.937 |
| 1/100 | 0.305 | 0.279 | 0.949 |

This assay will be useful for detecting the presence of rotavirus antibodies in blood, saliva, or fecal samples and may provide a rapid assay for determining rotavirus type specificity.

### Example 11

Isolates from recombinant baculovirus-gene 9 infected insect cells according to the present invention may be column purified and employed to produce antisera or as a vaccine.

Antisera may be specifically produced by immunizing rabbits with injections of purified recombinant VP7 protein according to the present invention as follows. The first inoculation contains the antigen with Freund's complete adjuvant.  Succeeding inoculations contain the antigen and Freund's incomplete adjuvant.  The animals are bled to obtain sera.  Polyclonal antibodies may be isolated from the sera by affinity chromatography.  These polyclonal antibodies may, for example, be utilized as reagents in a diagnostic assay for detection of rotavirus in a sample such as the Rotazyme[R] Immunoassay available from Abbott Laboratories, North Chicago, Illinois.

A passive vaccine may be produced by immunizing animals with injections of purified recombinant VP7 protein according to the present invention.  Polyclonal antibodies directed against subunit VP7 can be isolated from the serum, milk or other body fluid of the animal.  These may be further purified and used for therapeutic or prophylactic applications.

An active vaccine solution according to the present invention may be prepared by suspending the recombinant VP7 according to the present invention in an immunologically acceptable diluent, adjuvant and carrier.  Initial and booster injections or oral delivery of vaccine solution may be used to confer immunity.

Example 12

Monoclonal antibodies according to the present invention may be produced according to the procedure of Greenberg et al., J. Virol., 47, 267-275 (1983) with the substitution of a

solution of recombinant VP7 protein according to the present invention for the rotavirus concentrate employed therein. Greenberg, et al., is incorporated by reference herein.

Basically, monoclonal antibodies are produced by injecting mice with immunizing doses of recombinant VP7 protein, as described in Example 11. Spleens are removed from the immunized animals, and spleen cells are fused to myeloma cells (e.g. NS-1 cells) using polyethylene glycol. Hybridoma cells producing monoclonals are selected by screening in hypoxanthine aminopterin thymidine (HAT) medium. Monoclonal antibodies specific for VP7 recombinant protein may be isolated by affinity chromatography from media in which such hybridomas have been cultured.

Although the present invention is described in terms of a preferred embodiment, it is understood that modifications and improvements will occur to those skilled in the art. For example, according to the invention, a recombinant VP7 protein expressed in baculovirus infected insect tissue culture can be synthesized in large quantities at a low cost. Such a recombinant antigen could also be modified by site-specific mutagenesis providing a reagent with superior antigenic properties for diagnostic use or for vaccine use such as a high potency or broader spectrum vaccine.

Accordingly, it is intended that the appended claims include all such equivalent variations which come within the scope of the invention as claimed.

## CLAIMS

1.    A recombinant rotavirus outer capsid subunit protein produced in a baculovirus-insect cell expression system.

2.    The recombinant rotavirus subunit protein of Claim 1 wherein said protein is a major outer capsid protein (VP7).

3.    The recombinant rotavirus subunit protein of Claim 2 wherein said protein is a rotavirus protein specifically reactive with antisera against rotavirus serotype 3.

4.    The recombinant rotavirus subunit protein of Claim 3 wherein said protein is a rotavirus SA-11 protein.

5.    A nucleic acid encoding the recombinant subunit protein of claim 1 inserted into an E. coli-baculovirus shuttle vector.

6.    A recombinant subunit protein of the major outer capsid protein (VP7) of rotavirus SA-11 produced in a baculovirus-insect cell expression system, the recombinant subunit protein having a molecular weight of about 34 kilodaltons and being reactive with neutralizing antisera against rotavirus SA-11.

7.    An assay for detecting the presence of anti-rotavirus antibodies in a sample comprising the steps of:

exposing a sample containing the anti-rotavirus antibody to a recombinant subunit protein as recited in Claim 1, the protein being bound to a support;

introducing antibody specific for the anti-rotavirus antibody associated with a label; and

detecting the presence of the label bound to the support.

8.    A vaccine comprising an immunologically acceptable diluent, adjuvant or carrier and a recombinant subunit protein as recited in Claim 1.

9.    A method for raising a polyclonal antibody against a recombinant subunit protein as recited in Claim 1 comprising the steps of:

immunizing an animal with an effective amount of a recombinant subunit protein as recited in Claim 1; and

isolating anti-VP7 antibodies from serum, milk or other body fluid of the animal.

10.    A polyclonal antibody produced according to the method as recited in Claim 9.

11. A method of producing a monoclonal antibody against recombinant subunit protein as recited in Claim 1 comprising the steps of:

immunizing an animal with an effective amount of recombinant subunit protein as recited in Claim 1;

fusing antibody-producing cells from the animal with myeloma cells;

isolating a hybridoma in a culture medium producing a monoclonal antibody against VP7; and

purifying the monoclonal antibody from the culture medium.

12. A monoclonal antibody produced according to the method as recited in Claim 11.

13. A passive vaccine comprising the polyclonal antibody as recited in Claim 10.

14. A passive vaccine comprising the monoclonal antibody as recited in Claim 12.

30-48000

0251467

# Figure 1

# Figure 2

## RESTRICTION MAP OF SA-11 GENE 9 DNA CLONED INTO PstI SITE OF pUC9 (pAR91)

# Figure 3

## SEQUENCE OF SA-11 GENE 9
## CLONED DNA (pAR91)

```
                                                          36                                        67
GGCTTTAAAAAGAGAGAATTTCCGTTTGGCTAGCGGTTAGCTCCTTTTA ATG TAT GGT ATT GAA TAT
BOTH        -
ARIAS

                                      94                                        121
ACC ACA GTT CTA ACC TTT CTG ATA TCG ATT ATT CTA CTA AAT TAC ATA CTT AAA
                                          C

                            148                                        175
TCA TTA ACT AGA ATA ATG GAC TTT ATA ATT TAT AGA TTG CTT TTT ATA ATT GTG
                            G                             T

                        202                                        229
ATA TTG TCA CCA TTT CTC AGA GCA CAA AAT TAT GGT ATT AAT CTT CCA ATC ACA
                T                                                      G
                                256                                        283
GGC TCC ATG GAC ATT GCA TAC GCT AAT TCA ACG CAA GAA GAA ACA TTC CTC ACT
                    C                                            C
                    C
                                310                                        337
TCT ACA CTT TGC CTA TAT TAT CCG ACT GAG GCT GCG ACT GAA ATA AAC GAT AAT
                                    C

                                364                                        391
TCA TGG AAA GAC ACA CTG TCA CAA CTA TTT CTT ACG AAA GAG TGG CCA ACT GGA
                                                        G
                                                        G              A
                            418                                        445
TCC GTA TAT TTT AAA GAA TAT ACT AAC ATT GCA TCG TTT TCT GTT GAT CCG CAG

                            472                                        499
TTG TAT TGT GAT TAT AAC GTA GTA CTA ATG AAA TAT GAC GCG ACG TTG CAA TTG

                            526                                        553
GAT ATG TCA GAA CTT GCG GAT CTA ATA TTA AAC GAA TGG TTG TGT AAT CCA ATG
                                            T
                            580                                        607
GAT ATT ACT CTG TAT TAT TAT CAG CAA ACT GAC GAA GCG AAT AAA TGG ATA TCA

                            634                                        661
ATG GGC TCA TCA TGT ACA ATT AAA GTA TGT CCA CTT AAT ACA CAA ACT CTT GGA

                            688                                        715
ATT GGA TGC TTG ACA ACT GAT GCT ACA ACT TTT GAA GAA GTT GCG ACA GCT GAA
                                                              G C
                            742                                        769
AAG TTG GTA ATT ACT GAC GTG GTT GAT GGC GTT AAT CAT AAG CTG GAT GTC ACA

                            796                                        823
ACA GCA ACG TGT ACT ATT AGA AAC TGT AAG AAA TTG GGA CCA AGA GAA AAC GTA

                            850                                        877
GCC GTT ATA CAA GTT GGT GGT TCT GAC ATC CTC GAT ATA ACT GCT GAT CCA ACT

                            904                                        931
ACT GCA CCA CAG ACA GAA CGG ATG ATG CGA ATT AAC TGG AAA AAA TGG TGG CAA

                            958                                        985
GTT TTT TAT ACT GTA GTA GAC TAT GTA GAT CAG ATA ATA CAA GTT ATG TCC AAA

                            1012                                       1027
AGA TCA AGA TCA CTA AAT TCA GCA GCA TTT TAT TAC AGA GTG TAGGTATAACTTAGGTT

AGAATTGTATGATGTGACC
```

Figure 4
## CLONING OF TRUNCATED SA-11 GENE 9 IN AN NPV CLONING VECTOR